Europäisches Patentamt

European Patent Office

Office européen des brevets

(19)

(11) Publication number: **0 432 692 A1**

# EUROPEAN PATENT APPLICATION

(21) Application number: 90123693.5

(51) Int. Cl.5 **A61B 17/12**

(22) Date of filing: **10.12.90**

(30) Priority: **11.12.89 US 448471**

(43) Date of publication of application:
**19.06.91 Bulletin 91/25**

(84) Designated Contracting States:
**AT BE CH DE DK ES FR GB GR IT LI LU NL SE**

(71) Applicant: EDWARD WECK INCORPORATED
85 Orchard Road
Princeton New Jersey 05443-5251(US)

(72) Inventor: **Vernon, Paul M.**

101 Doar Road
Chapel Hill, NC(US)
Inventor: Phillips, John C.
4520 Briarglen Lane
Holly Springs, NC(US)
Inventor: Lee William
107 Legacy Lane
Durham, NC(US)

(74) Representative: Vossius & Partner
Siebertstrasse 4 P.O. Box 86 07 67
W-8000 München 86(DE)

(54) **Grip surgical clip.**

(57) The present invention is directed to a method of increasing the surface roughness or at least portions of surgical clips by applying coatings of material to the tissue gripping surfaces of hemostatic clips and/or to the outwardly directed faces of the blades of aneurysm clips. The material may include metal particles or ceramics. Preferable ceramics are alumina and even more preferable hydroxyapatite. The invention is also directed to the clips with such coatings applied thereto.

FIG. 7

## GRIP SURGICAL CLIP

This invention relates to surgical clips useful in occluding tubular members, more particularly, it is directed to hemostatic clips with an improved gripping mechanism for occluding vessels, such as blood vessels, and/or for securing the clips after surgery.

The use of metallic and polymer clips to occlude blood vessels during surgery are well known. There are many examples of patents covering hemostatic clips and aneurysm clips.

While metallic hemostatic clips have gained widespread acceptance, a major complaint is that the clips can be wiped or pulled off the vessels to which they are applied when using gauze wipes, etc. during surgery. Surfaces are generally fatty and wet and therefore very slippery. Clips can slip off or be pulled off the vessel even when properly applied:

Many clip designs have attempted to solve this problem, for example, see U.S. Patent No. 3,363,628 which discloses a longitudinal groove and cross serrations (coined in place) in the arms of the clip to stabilize or immobilize the clip on the vessel. While these clips are commercially successful, there is still a problem with the clips being pulled off during surgery.

SUMMARY OF THE INVENTION

It is an object of the present invention to provide improved surgical clips. ether object of the present invention is to improve the tissue gripping surfaces of surgical clips, more particularly, hemostatic clips.

Another object of the invention is to stabilize or immobilize aneurysm clips after application to a vessel.

The present invention is directed to a method of increasing the tissue gripping characteristics of surgical clips and the clips manufactured or provided thereby. The invention provides for increasing the surface roughness of at least the tissue gripping surfaces of the clip by fixedly applying a layer of material to the tissue gripping surfaces of a hemostatic clip. While the clips could be metallic or polymeric, in the preferred embodiment, the clips are metallic, for example stainless steel, titanium or tantalum. In one method, a coating of metallic particles is applied to the tissue gripping surfaces of the clip. The particles are stainless steel when a stainless steel clip is used or titanium when a titanium or tantalum clip is used. More specifically, the metallic particles are mixed together with a binder such as a polyvinylalcohol and methylcellulose and the combination brushed or sprayed or compacted onto the tissue gripping surfaces of the clip. The clip with the coating is then dried. Then the clip is heated to a first temperature, for example 300° C, to drive off the binder. Following this the coated clip is raised to the sintering temperature for the metal particles being used for a predetermined time period, e.g. anywhere from ten minutes to one hour depending on the density of the coating desired. The sintering temperature is about 1300° C for stainless steel and about 1100° C for titanium. Alternatively, the metallic particles may be glued onto the surface using a surgical glue such as methyl methacrylate.

Alternatively, a ceramic material is applied to the surface, such as alumina or hydroxyapatite. Where a ceramic or hydroxyapatite is used, a plasma spray or sputtering ion beam is used to apply the ceramic to the tissue gripping surfaces. It may be necessary when applying hydroxyapatite to subsequently heat the clip after the material has been applied.

When an aneurysm clip is coated, both the outwardly directed surfaces and the opposite tissue gripping surfaces of the arms which clamp the vessel may be coated. Alternatively, only the outwardly directed surfaces are coated but not the tissue gripping surfaces. Surrounding tissue will then grow through the spaces of the ceramic on the coated surfaces of the aneurysm clip to further immobilize the clip.

The ceramic material applied to the clips may also include biocompatible glass materials such as calcium phosphate silica glass.

BRIEF DESCRIPTION OF THE DRAWINGS

Figure 1 is a perspective view of a prior art hemostatic clip particularly suitable for use with the present invention.

Figure 2 is an elevational view of the clip shown in Fig. 1.

Figure 3 is a view of the clip taken along the lines and arrows 3-3 in Fig. 2.

Figure 4 is a cross-sectional view of the clip taken about the lines and arrows 4-4 in Fig. 2.

Figure 5 is a perspective view of a blood vessel with a prior art clip clamped thereon.

Figure 6 an elevational view of the clip of Figs. 1-4 with an improved gripping means in accordance with the present invention.

Figure 7 is a greatly enlarged longitudinal cross-section along the lines and arrows 7-7 in Fig. 6.

Figure 8 is a greatly enlarged cross-section of the clip of Fig. 6 taken along the lines and arrows 8-8 in Fig. 6.

Figure 9 is a perspective view of a blood vessel with an improved gripping clip in accordance with the present invention clamped thereon.

Figure 10 is a perspective view of the hemostatic clip of Fig. 1 with an alternate embodiment improved gripping means.

Figure 11 is a greatly enlarged cross-section of the clip of Figure 10 taken along the lines and arrows 11-11 in Fig. 10.

Figure 12 is a greatly enlarged cross-section of the clip of Fig. 10 shown clamped on a vessel.

Figure 13 is an elevational view of a prior art aneurysm clip with a coating attached to a portion thereof.

Figure 14 is a greatly enlarged top view of the aneurysm clip of Fig. 13.

Figure 15 is an enlarged cross-sectional view as taken on the line 15-15 of Fig. 13, showing the clip engaged with an aneurysm.

Figure 16 is a perspective view of a fixture used to hold a plurality of the clips of FIG. 1 which are to be coated to improve their gripping capability.

Figure 17 is a top planar view of the fixture of FIG. 16 without the clips.

Figure 18 is an end view of the fixture of FIG. 16 with one end removed.

Figure 19 is a schematic of an apparatus for applying an improved gripping coating to the wire which forms the clips of FIG. 1 before the clips are cut and fully formed.

## DETAILED DESCRIPTION OF THE DRAWINGS

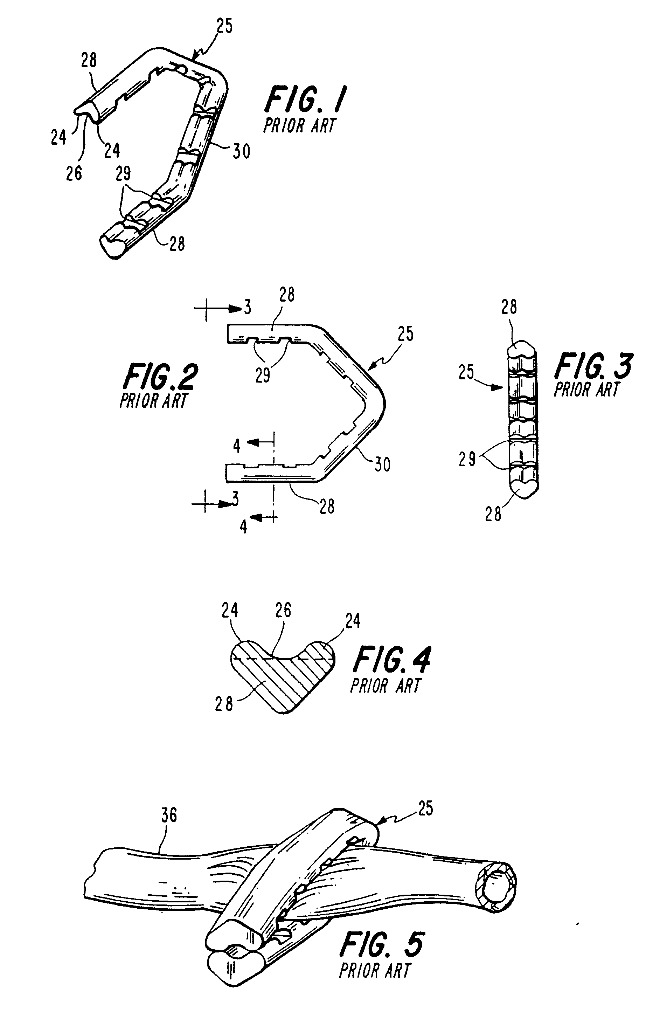

As illustrated in Figures 1-4, the prior art hemostatic clip comprises a clip 25 having a generally triangular cross-section. Preferably the clip is made of a malleable metal and includes arms 28 which are spaced apart and parallel from one another. The facing inner surfaces of the arms which correspond to the base of the generally triangular cross-section are formed intermediate their lateral edges with a valley or recess portion 26 extending lengthwise through at least the end portion of the arms and preferably through the entire length of the clip. It should be understood that while this clip is shown with a longitudinal groove throughout it could have no groove or several longitudinal grooves.

The longitudinal groove is formed by ridges 24 which are formed along the recess portion of the groove and our preferably formed to curvilinear shape. The inner surfaces of the arms 28 are preferably, but not necessarily, formed with longitudinally, spaced apart, cross-wise extending serrations 29 which extend continuously cross-wise of the clip from edge to edge.

While the clip is most likely a metallic material it should be a non-toxic material, such as, stainless steel, titanium, tantalum or even a plastic or like material, which materials are capable of being deformed and which retain the amount of deformation once they are fixedly clamped upon the blood vessels.

In the Figures, the bail portion 30 of the clip intermediate the spaced arms 28 is bent to a substantially V-shape or U-shape but it will be apparent that the portions intermediate the arms may be formed to other contours, such as rounded shapes or polygonal shapes.

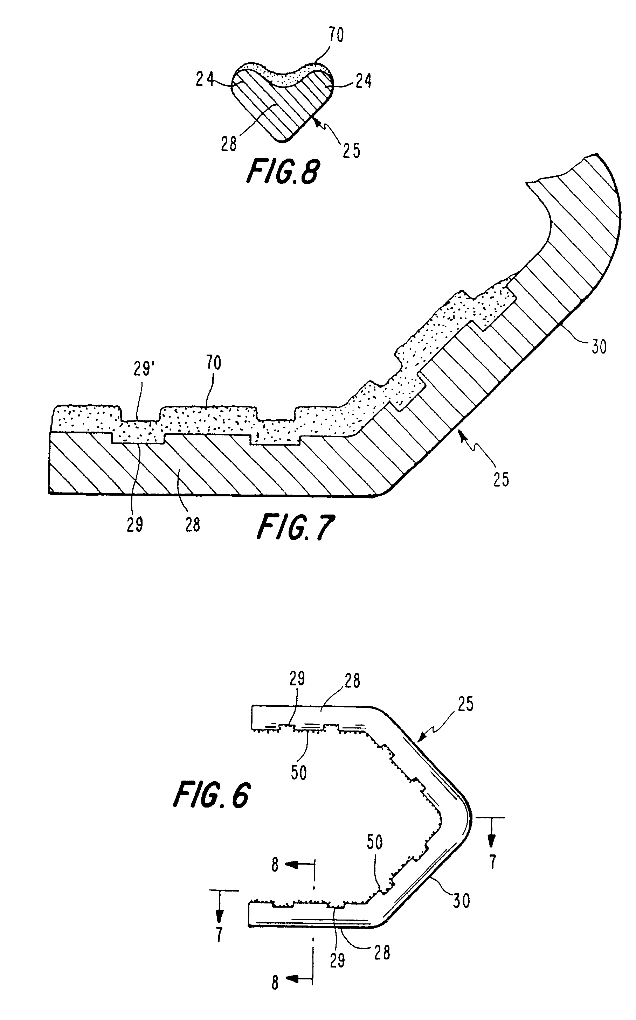

The depth and width of the recess 26 is not sufficient to noticeably weaken the clip, yet adequate to leave ridges 24 that will impress a circumferential band of compression about the blood vessel 36 in Figure 5 onto which the clip is attached. The cross-wise extending serrations 29 are dimensioned to have a depth and width which is substantially equal to the depth and width of the recess 26, and spaced to permit at least one and preferably more than one such serration to be in contact with the surface of the blood vessel upon which the clip is attached.

A clip applier and a cartridge for holding clips and making them accessible to the clip applier is described in more detail in U.S. Patent No. 3,363,628.

Despite the fine performance of the prior art clip as described in Figures 1-5, difficulties still arise with hemostatic clips on live blood vessels which are often coated with fluid and/or a fatty substance making them very slippery. Typically, during an operation a gauze wipe is used to clear away blood and other tissue from the surgical sight and threads of the gauze wipe can get caught up with the hemostatic clip pulling the clip off of the blood vessel. This is a serious problem for which many attempted solutions have been tried.

The present invention attempts to solve this problem by increasing the surface roughness of at least the tissue gripping surfaces of the clips by providing a coating of material on the tissue gripping surfaces of the hemostatic clip. Referring to Figure 6, the hemostatic clip, whose design is exactly the same as that shown in Figures 1-4 with like parts having like numerals thereon, includes a coating of material designated 50 in Figure 6. Figure 6 is a greatly enlarged elevational view and the actual hemostatic clip and the material 50 is shown greatly enlarged.

Several different materials can be used to coat the inner faces of the clip. For example, when using titanium or tantalum clips, titanium metal particles can be applied to the inner faces. A powder formulation of titanium or tantalum particles with specified particle sizes are mixed with a binder, for example polyvinylalcohol and methylcellulose. The coating is applied to the inner vessel gripping surfaces of the clip either by spraying or brushing or compacting onto the surface. The coating is dried at low temperature, for example, for 30 minutes under a heat lamp; the coated clips are then placed in a controlled atmosphere, such as a vacuum furnace or a furnace using inert gas, and then the clips are heated to a temperature where the binder is burnt off, for example, at $300^\circ$ C for an hour; and then the temperature of the coated clips is raised for a predetermined time period to the sintering temperature for the metal particles being used, e.g. to about $1300^\circ$ C for stainless steel or about $1100^\circ$ C for titanium. The predetermined time period can range from ten minutes or less to an hour or more. The longer the period the denser will be the coating.

Titanium powder with particle sizes in the range of 0.0010 inches in diameter to 0.0020 inches in diameter are recommended for sintering onto the inner surface of the hemostatic clip. The protrusions, such as the protrusions 50 in Figure 6, thus produced will tend to slightly penetrate the wall of the vessel being ligated helping to hold the clip in place. Such a clip could be applied with existing devices, including manual and automatic appliers. As an alternative way of applying the metallic particles, a surgical glue may be used, e.g. methyl methacrylate.

Materials other than metallic particles may be used to coat the vessel gripping surfaces of the clip in order to make the tissue gripping capability greater. Ceramics and biocompatible glasses are examples of such material. When applied to the tissue gripping surfaces, they provide a sand paper like surface which presents increased friction. Preferably, alumina or hydroxyapatite materials can be used. In particular, hydroxyapatite coatings would serve not only as an increased gripping surface but also as a retention surface on hemostatic and aneurysm clips. Animal studies have demonstrated that when placed in contact with soft tissue in vivo, hydroxyapatite will become attached by growth of connecting tissue on the surfaces and into the pores of the ceramic.

The ceramic coatings can be applied using a plasma spray which provides a controlled surface roughness on the clip gripping surface (approximately 12-25 microns in thickness) and increase the frictional contact with tissue and thus aid in gripping the vessel during surgery. With respect to hydroxyapatite, tissue in-growth into the coating may help to permanently fix the clip in place preventing any post operative movement. A plasma spray could also be used for applying metal particles to metal clips. Alternatively, a physical vapor deposition process might be used to apply the ceramic or metal coating to the clip surface. With this process, coatings will be quite thin, e.g. one or two microns or less.

Figure 7 is a greatly enlarged cross-sectional view of the clip of Fig. 6 to which a coating has been applied. In Fig. 7 the coating layer is indicated as layer 70. Particularly when using a plasma spray this layer will be more continuous than the discrete particles shown in FIG. 6, but the surface will still be rough with greater gripping ability. The coating 70 as mentioned may be in the range of about 12-25 microns thick and can comprise alumina or hydroxyapatite or other ceramics. Other thicknesses both thinner and thicker might also be used. Ten to twelve microns seems quite suitable for hemostatic clips while twenty-five microns has been used with aneurysm clips. It will be noticed that the layer 70 follows the contours of the serrations 29 in region 29'. While it is shown that the coating layer extends along the bail portion of the clip it may be preferable only to apply the coating to the leg or arm 28 tissue gripping surfaces.

An added step ensuring a good chemical bond between the coating (e.g. hydroxyapatite or titanium particles) and the metal comprises applying heat to the coated clips after the plasma spray is finished. In plasma spraying, the material is heated up close to the melting point and when it impacts on the clip, it forms a chemical bond. This chemical bond may be aided by heating the coated clip after the coating is finished, e.g. in the case of titanium by heating in a vacuum from $900^\circ$ C to $1000^\circ$ C for 15 minutes to one hour.

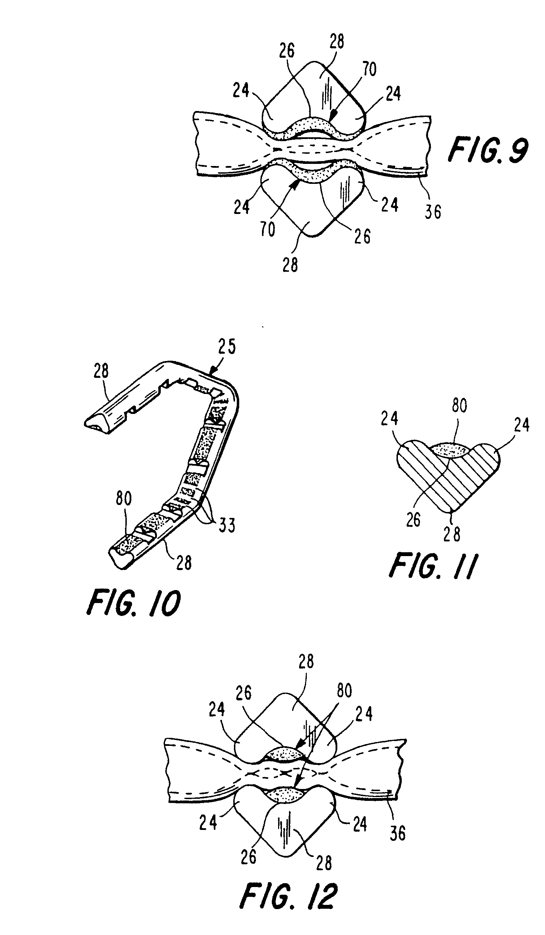

An alternate embodiment of coating the clip of Figure 1 is shown in Figs. 10-12, where a coating 80 is applied only within the longitudinal groove 26. The coating 80 remains even with or slightly below the peak of ridges 24 which are on either side of the longitudinal groove 26. Fig. 11 is a cross-section through the leg of Fig. 10 and demonstrates the level of the coating below the top of the groove. Fig. 12 is an end view of the clip 25 with coating 80 after it has been clamped about the vessel. Here the coating is clearly seen in contact with the vessel and yet the longitudinal groove still has the intended effect of helping to keep the attached clip on the vessel. In the long term, where the coating is hydroxyapatite, tissue is able to grow into the coating and eventually creates a tighter bond with the clip so that it will not be dislodged. This could be particularly important when a patient with internal clips is subjected to an MRI scan where the strong magnetic fields can actually move the stainless steel clips. In particular, titanium is used because it has less reactance to the magnetic field and is less likely to move. When combined with a hydroxyapatite coating which includes frictional grab and stimulates growth of the tissue within the spaces in the hydroxyapatite ceramic, it is expected that little or no movement will result with the titanium clip with the hydroxyapatite coating.

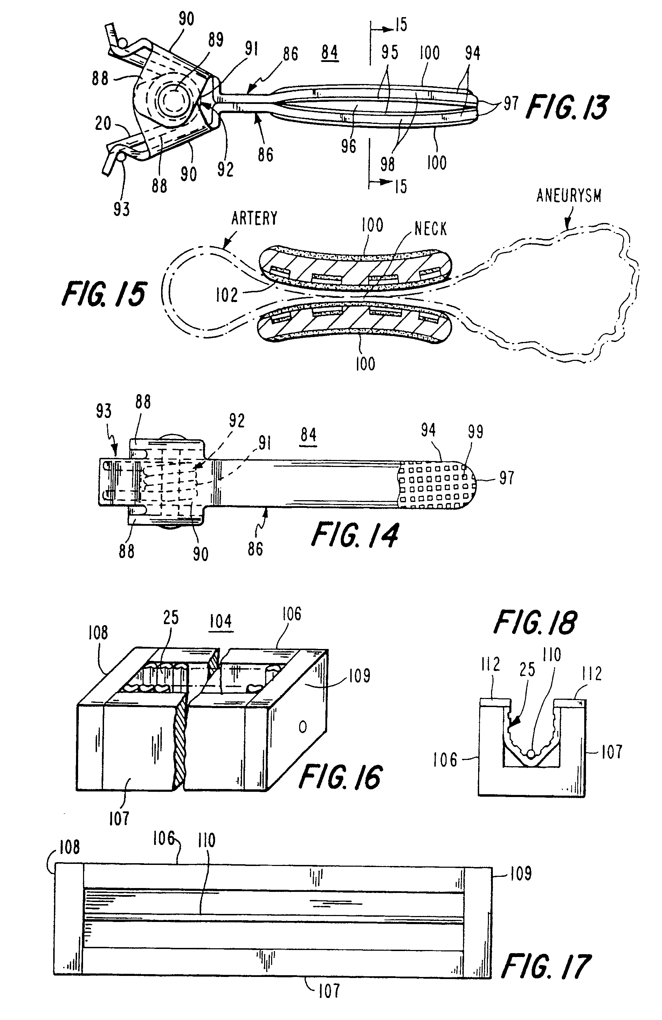

Figure 13 is an example of an aneurysm clip designated generally 84. Details of the aneurysm clip can be found in U.S. Patent No. 3,598,125. There are many other examples of aneurysm clips. The aneurysm clip comprises clamping members 86 which are provided with ears 88. A pivot pin 89 extends through aligned holes of the ears 88 with the ear of one member being outside the ear of the other. The ends of the pin 89 are staked or flattened over the outer faces of the outer ears. The ears 88 are bent from flat arm portions 90 of the arms 86 which span across a space in which the coils 91 of the spring 92 reside. The proximal end of each clamping member 86 comprises a hook 93 formed in a concave shape of generally V-shaped form and extending from the flat portion 16. The hooks are in opposed relation, their apices contacting each other when they are pressed toward each other to cause the arm ends to spread apart. The proximal end of each clamping member is formed with a notch not shown to receive an end of the spring 92, the notches and spring ends being offset. The spring ends are normally biased apart to move the arms 94 to the closed position as shown in Fig. 13.

The arms 94 on members 86 are preferably oppositely, longitudinally bowed at their intermediate portions 95, the same defining an opening 96 between them when the distal ends 97 are in contact, as in Fig. 13, under normal bias of the spring ends on the hook ends 93 of members 10. In addition to the arms being bowed, the same are transversely curved so only the middle longitudinal portions of the distal ends 97 are in contact with each other. The middle longitudinal portions of the bowed portions are closer together than are the longitudinal edges 98 of the bowed portions as seen in Fig. 13.

Unlike ligating clips, it is important that the interfaces or the tissue contacting faces of the arms 94 on aneurysm clips, have firm yet non-abrading engagement with the outer wall of an aneurysm. Surfaces are provided with a multiplicity of depressions 99 that are defined by intersecting portions of said surfaces. A coating 100 of hydroxyapatite is applied to the opposite outer surfaces of the arms as depicted in Figs. 13 and 15. When dealing with aneurysms, it is very important that the clip remain in place long after the operation is concluded. Since the hydroxyapatite will readily receive tissue growth and allow tissue growth to be intertwined between the spaces formed in the ceramic, the hydroxyapatite is placed on the outside surfaces of the arms 94 to receive the tissue and firmly hold the clamp in place. It is also possible, but not necessary, to apply a relatively smooth coating 102 of hydroxyapatite to the tissue gripping surfaces of the aneurysm clip as shown in Fig. 15.

While particular metallic clips, such as stainless steel, titanium and tantalum have been discussed with regard to the Figures, any suitable metals used for surgical clips could undergo the same type of coating operation. Similarly, the ceramics specifically called out, alumina and hydroxyapatite, are not the only ceramics that might be applied to clips in the fashion herein described.

Figs. 16-18 show one example of a fixture which is suitable for use in coating the tissue gripping surfaces of formed hemostatic clips. The fixture is designated generally 104 and comprises a metal block having a rectangular cross-section and being longer than it is wide. It comprises two spaced apart walls 106 and 107 which define a longitudinal rectangular channel 105 therebetween. The width of the channel accommodates a stacked column of hemostatic clips like the clip 25 shown in Fig. 1.

The fixture 104 also includes rectangular plates of metal 108 and 109 attached to the ends of the side walls 106 and 107. The clips are stacked in the channel such that the ends of the arms 28 of the clips are facing toward the open side of the channel while the bail portion 30 of the clip rests on the bottom of the channel.

To keep the clips securely held in place, a cylindrical metal bar 110 is inserted through an opening in the end panel walls 108 and 109 near the bottom of the channel. The bar is inserted after all of the clips have been placed in the channel and the bar engages the interior V-shaped portion of the bail of the clips. A plasma spray or physical vapor deposition beam is aimed at the interior surfaces of the legs of the clip through the open top of the channel in the fixture. The angle of the plasma spray or physical vapor deposition beam is transverse to the plane which bisects the channel through the open end of the channel. This provides a more or less even coating on the interior surfaces of the legs and prevents the coating from accumulating in the bail portion of the clip.

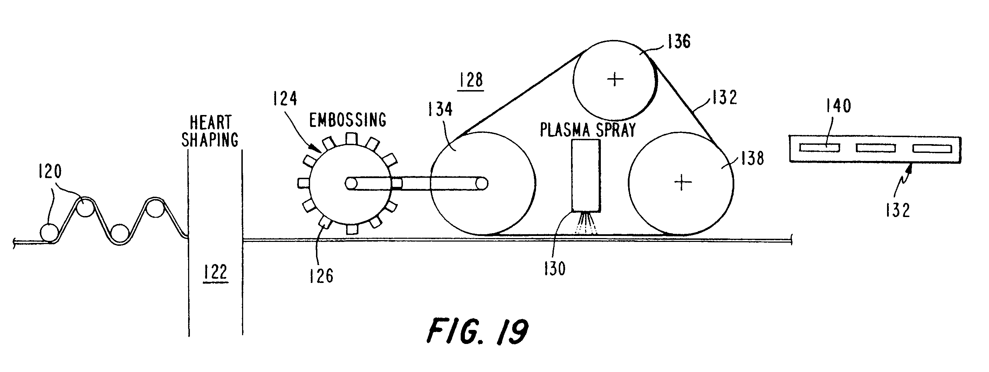

Fig. 18 is an end view of the fixture of Fig. 16 with one of the panel walls removed. It shows the positioning of the retaining rod 110 relative to hemostatic clips 25 and the positioning of the clips within the channel 105 of the fixture. In addition, metal plates 112 may be attached to the top surfaces of the channel walls 106 and 107. The plates 112 extend partially over the channel 105 and overlap the end faces of the legs of the clips. This masks the end faces of the legs of the clips from the coating spray or beam. With the fixture 104 of Figs. 16-18, a coating layer can be applied to the inner surfaces of the hemostatic clips while masking the end faces of the clips and the interior portion of the bail of the clips.

Fig. 19 is a schematic diagram of an apparatus for applying a coating, such as by plasma spray, to the hemostatic clip wire before the wire is cut and the wire pieces formed into individual clips. Wire enters the apparatus from the left where it is straightened through a series of rollers 120 and then moves through a heart shaping station 122. The heart shaping station comprises a series of rollers and the longitudinal groove is coined in place. As the heart shaped wire proceeds through the apparatus, it moves under the embossing wheel 124 which impresses the serrations across the longitudinal groove into the regions 24 of the heart shaped wire. The embossing wheel comprises a series of hard metal protrusions 126 which are equally spaced apart and do not extend completely around the circumference of the wheel. This leaves a space on the wheel larger than the spacing between adjacent protrusions. This space corresponds to the apex of the bail portion 30 of the hemostatic clip when the clip is formed.

The wire, after passing through under the embossing wheel 124, passes by the coating station 128. A source of coating material such as a plasma spray 130 is directed toward the longitudinal groove of the heart shaped wire. A slotted band 132 passes around rollers 134, 136 and 138 to pass between the plasma spray and the heart shaped wire. The drive wheel 134 for the coating station is slaved to the embossing wheel to rotate in synchronization therewith. The slotted band comprises a series of rectangular slots 140. Where ever the slot occurs the plasma spray or coating will pass through the slot onto the heart shaped wire. The remaining part of slotted band masks the heart shaped wire from the coating spay. The slots are shaped and spaced to lay a desired pattern of coating on the wire such that when the wire is cut into the clips and the clips are formed, the coating will be strategically placed on the desired surfaces of the clip.

The slots could be shaped so as to provide a coating across the entire tissue gripping surface of the legs 28 of Fig. 1 in a pattern like that shown in Figs. 7 and 8. Alternatively, the slots can be formed to allow a coating to be placed only in the longitudinal groove 26, but not across the peaks of the regions 24 as shown in Fig. 11.

It may be desirable to interrupt the coating along the longitudinal groove of the clip as shown by the interruptions 33 in Fig. 10. When the clips are first formed or when the clips are applied, certain regions of the wire undergo certain stress which may cause portions of the coating to loosen or flake off of the clip. This may be avoided by eliminating the coating from the regions of the stress when the wire is cut before the clips are formed. The apparatus of Fig. 19 using the slotted band in synchronization with the embossing wheel can be used to apply the appropriate pattern of interruptions to the coating applied to the clips.

## Claims

1. A method of increasing the tissue gripping characteristics of a surgical clip comprising the step of:

   increasing the surface roughness of at least the tissue gripping surfaces of the clip by fixedly attaching a layer of material on at least the tissue gripping surfaces of the clip.

2. The method of claim 1 wherein the clips are metallic clips and the material comprises metallic particles.

3. The method of claim 2 wherein the method comprises:

   applying a mixture of metallic particles and a suitable binder to the tissue clamping surfaces of the clip;

   drying the coating on the clip at a low temperature;

   heating the coated clip to a temperature where the binders burn off for a pre-determined period of time; and

   then raising the temperature to the sintering temperature for the metals being used.

4. The method of claim 1 wherein the material comprises a ceramic material.

5. The method of claim 2, 3, or 4, wherein the step of fixedly attaching comprises spraying said clips with a plasma spray.

6. The method of claim 2, 3, or 4, wherein said step of fixedly attaching comprises attaching said layer with a physical vapor deposition process.

7. The method Of claim 2, 3, or 4, wherein said step of fixedly attaching comprises attaching said layer by glueing with a surgical glue.

8. The method of claim 5 cross-referenced to claim 2 or 3, wherein said clips comprise titanium and said particles comprise titanium particles and said step of spraying with a plasma spray includes spraying in a low pressure or inert gas chamber.

9. The method of any one of claims 1 to 8, wherein the method further comprises heating the coated clips after the layer has been applied.

10. The method of any one of claims 5, 6 or 8, wherein said method further comprises masking portions of the clip to avoid coating said masked areas.

11. The method of claim 9 or 10, wherein the coating along the tissue gripping surfaces of the clip is interrupted in predetermined locations to avoid chipping of the coating from the clip when the clip is formed or applied.

12. A hemostatic clip comprising a pair of arms interconnected at one end and opened at the other with the arms arranged in laterally spaced apart substantially parallel relation, each of said arms having a side facing inwardly, said hemostatic clip further comprising a layer of material on at least said inwardly facing sides of said arms which increases the surface roughness of the tissue gripping surfaces of the clip.

13. The clip of claim 12 wherein said material comprises metal particles.

14. The clip of claim 13 wherein said clip comprises stainless steel and said metal particles comprise stainless steel.

15. The clip of claim 13 wherein the clip comprises titanium or tantalum and said metal particles comprises titanium.

16. The clip of claim 12 wherein said material comprises a ceramic.

17. The clip of claim 16 wherein said ceramic comprises hydroxyapatite.

18. The clip of claim 16 wherein said ceramic comprises alumina.

19. The clip of claim 16, 17 or 18, wherein said ceramic comprises a biocompatible glass material.

20. The clip of any one of claims 15 to 19, wherein each of the inwardly facing sides of said arms comprises at least one longitudinal groove and at least one cross-wise serration.

21. The clip of claim 20, wherein each of said arm portions is generally triangular in cross-section with the apex of said triangular cross-section facing outwardly in the opposite direction from said inwardly facing side.

22. The clip of claim 20 or 21, wherein said material is applied only within said longitudinal groove.

23. An aneurysm clip comprising:
a pair of clamping arms having proximal and distal ends and having opposed clamping faces and opposite outwardly directed faces, said arms being pivoted intermediate said ends and adapted for clamping engagement with the outer surfaces of the walls of an arterial vessel;
spring means resiliently biasing said arms into clamping position; and
a layer of ceramic material on the opposite outwardly directed faces of said clamping arms, said ceramic material disposed to accept tissue growth from the surrounding tissue.

24. The aneurysm clip of claim 23 wherein said clamp comprises a layer of ceramic material on said opposed clamping faces.

25. The aneurysm clip of claim 23 or 24 wherein said ceramic material comprises hydroxy-apatite.

26. A method of stabilizing an aneurysm clip having a pair of clamping arms with opposed clamping faces and opposite outwardly directed faces comprising the step of fixedly applying a layer of ceramic material to the oppositely, outwardly directed faces.

27. The method of claim 26 wherein said method further comprises applying a layer of ceramic material to said opposed clamping surfaces.

28. The method of claim 26 or 27 wherein said ceramic material comprises hydroxyapatite.

FIG. 1
PRIOR ART

FIG. 2
PRIOR ART

FIG. 3
PRIOR ART

FIG. 4
PRIOR ART

FIG. 5
PRIOR ART

FIG.8

FIG.7

FIG.6

FIG. 9

FIG. 10

FIG. 11

FIG. 12

*FIG. 13*

*FIG. 15*

*FIG. 14*

*FIG. 16*

*FIG. 18*

*FIG. 17*

11

FIG. 19

12

European
Patent Office

**EUROPEAN SEARCH
REPORT**

Application Number

**EP 90 12 3693**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-3 631 858 (ERSEK) <br> * Column 2, lines 56-63; figure 1 * | 1-5,8, 12-19, 23-28 | A 61 B 17 12 |
| Y | US-A-4 784 159 (SZILAGYI) <br> * Claims 1,11 * | 1-3,5,8, 12-15 | |
| Y | US-A-4 737 411 (GRAVES) <br> * Column 6, lines 20-34 * | 4,16,19, 23,24,26, 27 | |
| Y | WO-A-8 808 460 (VENT-PLANT) <br> * Claim 1 * | 17,18,25, 28 | |
| A | WO-A-8 001 752 (LeVEEN) <br> * Page 3, lines 6-14; figure 1 * | 1,12 | |
| A,D | US-A-3 363 628 (WOOD) <br> * Claim 1 * | 12,20,21 | |
| A,D | US-A-3 598 125 (COGLEY) <br> * Claim 1 * | 23 | TECHNICAL FIELDS SEARCHED (Int. Cl.5) <br><br> A 61 B <br> A 61 F |

The present search report has been drawn up for all claims

| Place of search | Date of completion of search | Examiner |
|---|---|---|
| The Hague | 12 March 91 | MOERS R.J. |